# EUROPÄISCHE PATENTSCHRIFT

(11) **EP 0 300 131 B1**
(45) Veröffentlichungstag und Bekanntmachung des Hinweises auf die Patenterteilung: **20.10.1993**
(21) Anmeldenummer: 88104296.4
(22) Anmeldetag: 18.03.1988
(51) Int. Cl.: A61F 2/30, A61F 2/36

(54) **Sattelprothese**
Saddle prosthesis
Prothèse en forme de selle

(30) Priorität: 27.03.1987 DE 3710233
(43) Veröffentlichungstag der Anmeldung: 25.01.1989
(73) Patentinhaber: GMT Gesellschaft für medizinische Technik mbH, D-22767 Hamburg (DE); Waldemar Link (GmbH & Co.), D-22315 Hamburg (DE)
(72) Erfinder: Keller, Arnold, D-2061 Kaihude (DE); Nieder, Elmar, D-2155 York (DE)
(74) Vertreter: Heldt, Gert, Dr. Dipl.-Ing.

(56) Entgegenhaltungen:
- EP-A- 0 073 057
- EP-A- 0 079 441

## Beschreibung

Die Erfindung betrifft eine Sattelprothese zum Ersatz eines Hüftgelenkes mit zwei einen Sattel zwischen sich ausbildenden Sattelhörnern, die mit einem in einen Oberschenkelknochen einzusetzenden Schaft verbunden sind, wobei eine sich im wesentlichen in Richtung einer aus einer Gewichtskraft und einer Muskelkraft ausgebildeten Kraftresultierenden erstreckende Sattelquerachse versetzt zu einer sich im wesentlichen in lotrechter Richtung erstreckenden Schaftlängsachse angeordnet ist.

Obwohl mit einer solchen Sattelprothese, die aus der EP-A-0 079 441 und der entsprechenden deutschen Prioritätsanmeldung P 3 138 848 bekannt geworden ist, gute Erfahrungen gesammelt worden sind, stellt sich anhand einiger Implantationsfälle, in denen diese vorbekannte Sattelprothese eingesetzt worden war, heraus, daß eine Verbreiterung der Auflagefläche, auf der sich zwischen den Sattelhörnern der Beckenknochen abstützt, sehr wünschenswert sei. Insbesondere beim Einsatz der Sattelprothese an solchen Stellen des Beckenknochens, an denen dieser eine relativ dünne Wandstärke besitzt, muß damit gerechnet werden, daß die relativ schmale Auflagestelle, auf der sich der Beckenknochen der Sattelmulde abstützte, zu Verformungen der Knochenschmalstelle im Bereich des Beckenknochens führte. Darüber hinaus konnte nicht mit der notwendigen Sicherheit ausgeschlossen werden, daß die beiden Sattelhörner bei der Ausführung von Schwenkbewegungen des Oberschenkelknochens an benachbarte Teile des Beckenknochens anschlugen. Dadurch konnten in einigen Fällen lediglich Beschränkungen der Schwenkweite des Oberschenkels dazu führen, daß die Sattelhörner darin behindert wurden, an den benachbarten Beckenknochen anzuschlagen.

Aufgabe der vorliegenden Erfindung ist es daher, die Sattelprothese der aus der Entgegenhaltung bekannt gewordenen Art so zu verbessern, daß sie sich flexibel dem jeweiligen Bedarf eines Patienten anpassen kann.

Diese Aufgabe wird erfindungsgemäß dadurch gelöst, daß zwischen dem Schaft und dem Sattel ein den Schaft mit dem Sattel verbindendes Mittelstück angeordnet ist, mit dem der Sattel um die quer zum Sattel verlaufende Sattelquerachse drehbar verbunden ist.

Da sich nunmehr der Sattel gegenüber dem Mittelstück verschwenkbar der jeweiligen Schwenkstellung des Oberschenkels entsprechend einstellen kann, ist die Sattelmulde gegenüber dem auf ihr aufliegenden Teil des Beckenknochens relativ in Ruhe, so daß über diesen Teil des Beckenknochens im wesentlichen nur noch Druckkräfte auf den Sattel übertragen werden müssen, nicht jedoch Drehmomente, die ein Verschwenken der Sattelmulde gegenüber dem auf ihr aufliegenden Beckenknochen notwendig machen. Damit bildet sich im Laufe der Zeit eine bevorzugte Stellung der Ankopplungsstelle des Beckenknochens gegenüber der Sattelmulde heraus, die zusätzlich dadurch stabilisiert werden kann, daß die Sattelmulde im Bereich der Auflagestelle relativ breit ausgebildet werden kann. Durch diese Ausbildung der Sattelmulde wird zusätzlich der Druck auf die Ankopplungsstelle des Beckenknochens minimiert. Im Hinblick auf diese im Bereich der Ankopplungsstelle stark herabgesetzte mechanische Beanspruchung wird der Beckenknochen im Bereich der Ankopplungsstelle dazu angeregt, Knochenmasse anzulagern und damit eine relativ breite Auflagestelle im Bereich der Sattelmulde auszubilden. Je breiter diese Auflagestelle des Knochens wird, umso geringer ist die spezifische Druckbeanspruchung, die auf den Knochen einwirkt. Eine Verformung des Knochens wird in den weitaus meisten Fällen ausblieben, so daß mit einem nachträglichen Ersatz der aufeinander wirkenden Teile nicht gerechnet zu werden braucht. Der Träger der Sattelprothese behält sein übliches Gangbild, auf das die Sattelprothese bei ihrem Einsetzen vom Chirurgen eingestellt worden ist. Darüber hinaus kann mit einer hohen Standfestigkeit der Ankopplung gerechnet werden, so daß die Sattelprothese insgesamt mit der Aussicht auf eine lange Standzeit implantiert werden kann.

Gemäß einer bevorzugten Ausführungsform der Erfindung ist das Mittelstück als ein separates Bauteil ausgebildet. Diese separate Ausbildung des Mittelstückes hat den Vorteil, daß durch verschiedene Ausformungen des Mittelstückes der Chirurg die Möglichkeit hat, die Sattelprothese einer Vielzahl von Einsatzfällen problemlos anzupassen. Je nach Art der vorgefundenen anatomischen Ausbildung beim Einzelpatienten kann ein Mittelstück mehr oder minder großer Krümmung Verwendung finden. Außerdem kann das Mittelstück je nach Einsatzfall relativ lang oder weniger lang ausgebildet werden. Dadurch ist es möglich, den Schaft und den Sattel in weiten Grenzen nach einer vorgegebenen Norm auszubilden, um die Anpassung dieser beiden Bauteile mit Hilfe des separaten Mittelstückes vorzunehmen.

Gemäß einer anderen bevorzugten Ausführungsform der Erfindung ist der Sattel mit dem Mittelstück durch einen sich im wesentlichen in lotrechter Richtung erstreckenden Zapfen verbunden. Dieser Zapfen ermöglicht die Verwendung von Distanzstücken, die zwischen dem Mittelstück und dem Sattel eingesetzt werden und während der Operation eine problemlose Längenanpassung an die konkret vorliegende Situation erlauben. Vor Beginn der Operation kann nur sehr schwer abgeschätzt werden, welche Längen die einzelnen Elemente der Sattelprothese aufweisen müssen. Dieses resultiert daraus, daß meist nicht genau vorauszusehen ist, wie groß der Knochenverlust im Bereich des Beckenknochens tatsächlich ist. Durch die Verwendung der Distanzstücke kann während der Operation nach einem Einsatz des Schaftes und nach einer Anpassung des Sattels die erforderliche Längenanpassung in einfacher Weise durch seitliches Einschieben einer erforderlichen Anzahl von Distanzstücken bzw. von einem Distanzstück erforderlicher Dicke vorgenommen werden. Der Zapfen wird dazu so weit aus dem Mittelstück herausgezogen, daß dem Distanzstück kein Widerstand entgegengesetzt wird. Nach dem Einsatz eines entsprechend dimensionierten Distanzstückes wird die Längsachse durch das Distanzstück hindurch wieder in den Sattel hineingeschoben und damit die gewünschte Verbindung wieder hergestellt. Diese optimale Längenanpassung führt dazu, daß die Luxationsgefahr deutlich verringert werden kann.

Gemäß einer weiteren bevorzugten Ausführungsform der Erfindung ist das Mittelstück bezüglich seiner Ausdehnung in Richtung einer sich aus Muskel- und Gewichtskraft zusammensetzenden Kraftresultierenden an die konkrete vorliegende operative Situation anpaßbar ausgebildet. Eine derartig konfigurierbare Ausbildung weist den Vorteil auf, daß eine Anpassung an verschiedene operative Situationen vorgenommen werden kann, ohne daß Distanzstücke verwendet werden müssen. Durch Verwendung weniger Teile kann hierdurch eine äußerst stabile Ausbildung der Prothese erreicht werden.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

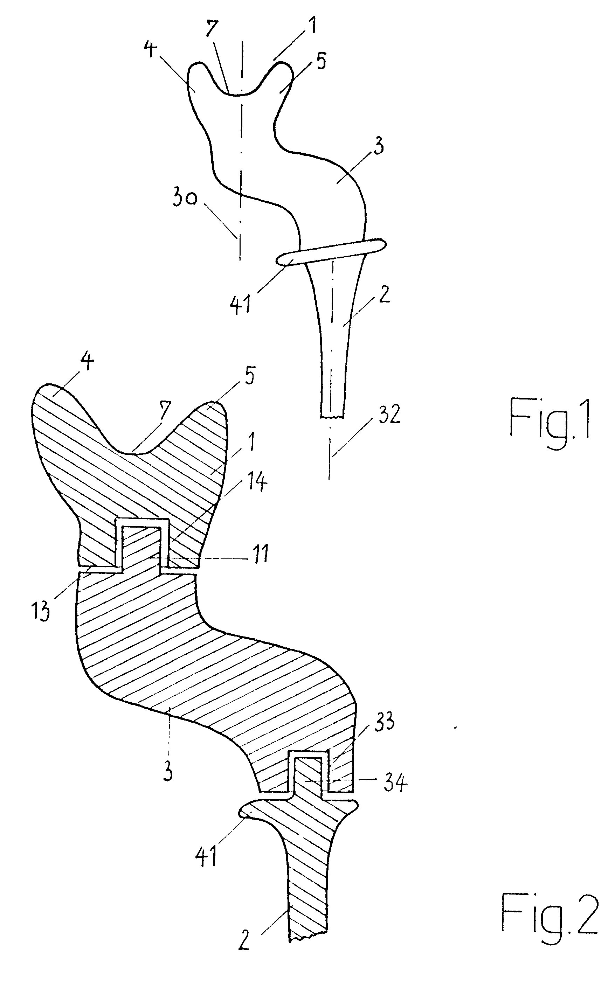

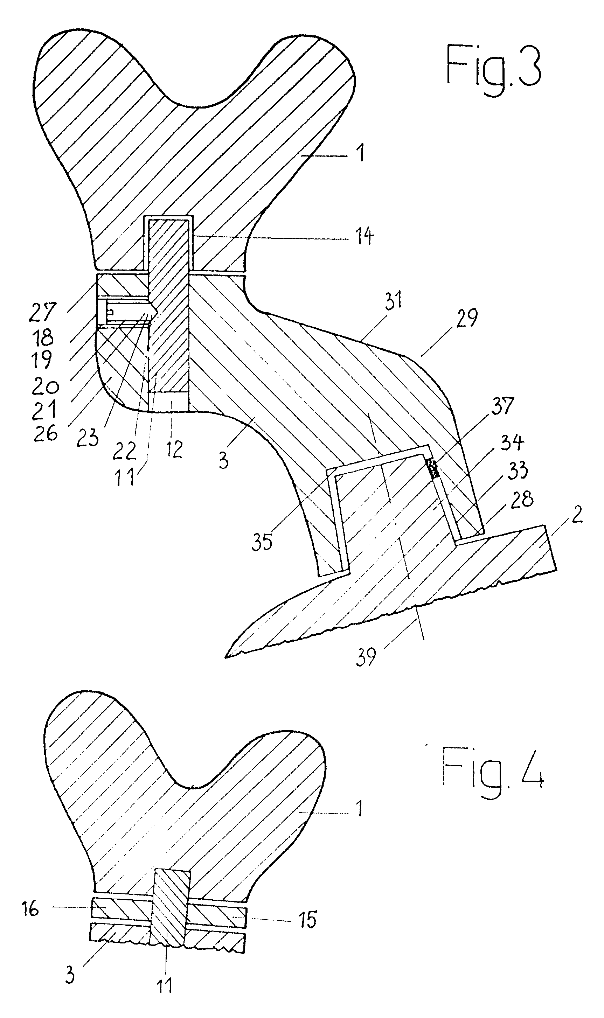

- Fig. 1:: eine Prinzipskizze einer Sattelprothese mit gegenüber einer Schaftlängsachse versetzter Sattelquerachse,
- Fig. 2:: eine im wesentlichen vertikalen Längsschnitt durch eine Sattelprothese mit gegeneinander beweglichem Sattel, Mittelstück und Schaft,
- Fig. 3:: einen Längsschnitt durch eine Sattelprothese, bei der sich durch das Mittelstück in Richtung auf den Sattel ein diesen führender Zapfen erstreckt,
- Fig. 4:: eine teilweise Darstellung eines Längsschnittes durch eine Sattelprothese, bei der zwischen dem Mittelstück und dem Sattel ein Distanzstück angeordnet ist,
- Fig. 5:: einen Längsschnitt durch einen Schaft, der in einen Oberschenkelknochen eingesetzt ist und einen zur Halterung des Mittelstückes vorgesehenen Konus aufweist,
- Fig. 6:: eine teilweise Darstellung eines Längsschnittes durch einen Schaft, der im Bereich seines in lotrechter Richtung oberen Endes einen Reimersverschluß aufweist, der über eine Spannschraube mit dem Schaft verbunden ist,

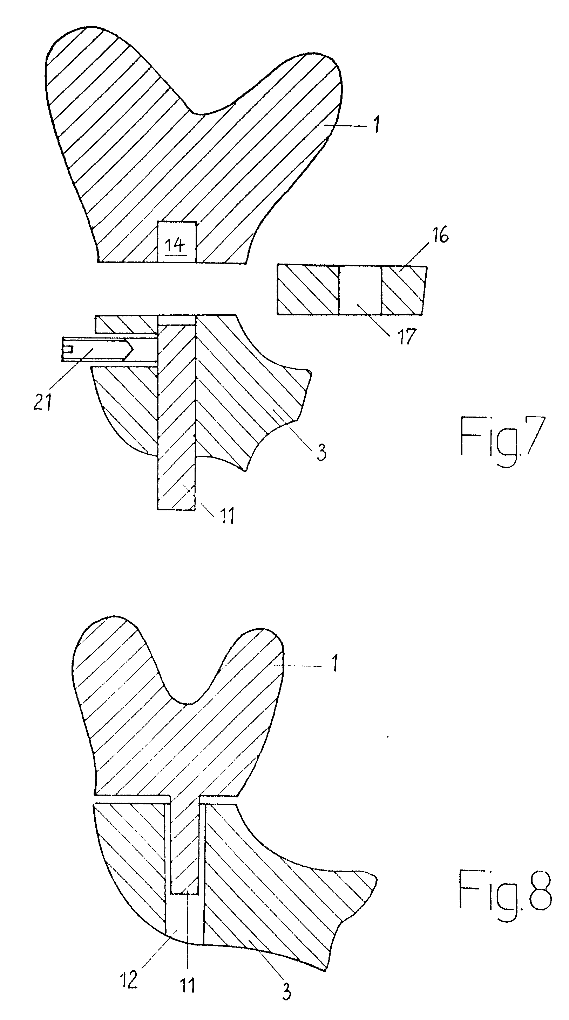

- Fig. 7:: eine teilweise Darstellung eines Längsschnittes einer Sattelprothese mit gelöstem, das Mittelstück mit dem Sattel verbindenden Zapfen und herausgezogenem Distanzstück,
- Fig. 8:: eine teilweise Darstellung eines Längsschittes einer Sattelprothese mit einem Sattel, der fest mit einem ihn im Bereich des Mittelstückes lagernden Zapfen verbunden ist,

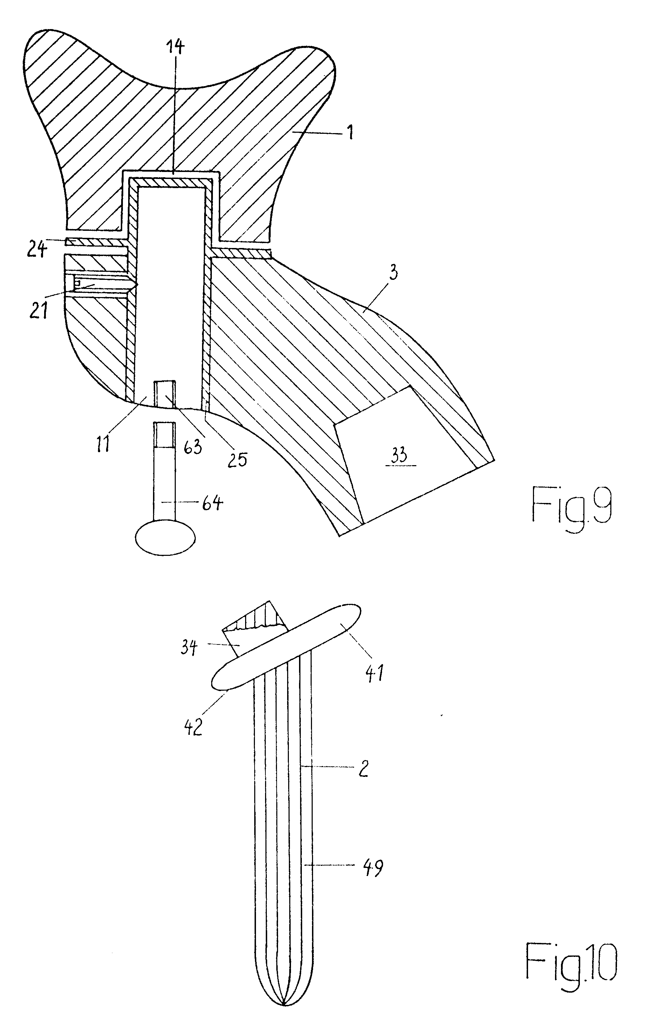

- Fig. 9:: einen Längsschnitt durch einen Sattel und ein Mittelstück, bei dem sich zwischen dem Sattel und dem Mittelstück eine Lagerschale erstreckt und bei dem der Zapfen im Bereich des Mittelstückes in einer Lagerbuchse geführt ist,
- Fig. 10:: eine Seitenansicht eines Schaftes mit sich in Richtung der Schaftlängsachse erstreckenden Längsnuten,

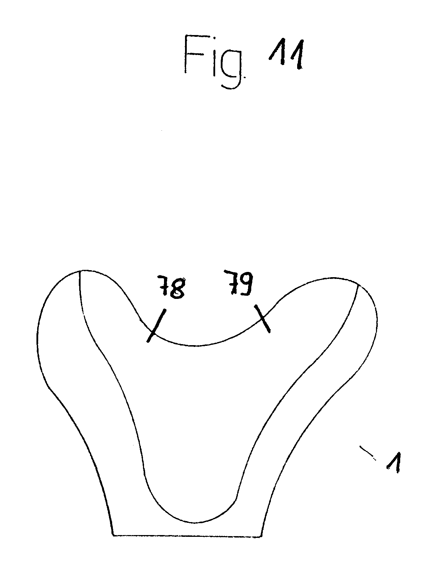

- Fig. 11:: eine Seitenansicht eines Sattels mit teilweise die Reibung herabsetzender Beschichtung und

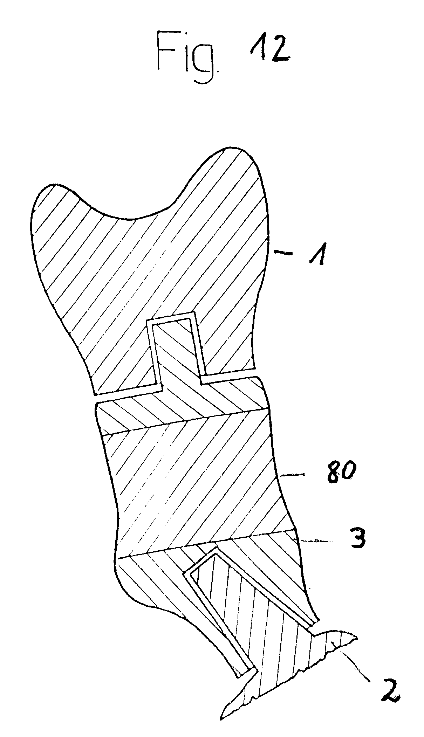

- Fig. 12:: eine teilweise Darstellung eines Längsschnittes durch eine Sattelprothese mit Dämpfungselement.

Eine Sattelprothese besteht im wesentlichen aus einem Sattel (1), einem Schaft (2) und einem Sattel (1) mit dem Schaft (2) verbindenden Mittelstück (3). Der Sattel (1) weist zwei Sattelhörner (4, 5) auf, die eine einen Beckenknochen halternde Sattelmulde (7) begrenzen. Der Beckenknochen liegt im Bereich einer Beckenmulde auf der Sattelmulde (7) auf. Die Sattelhörner (4, 5) erstrecken sich mindestens in einem Teilbereich ihrer Ausdehnung den Beckenknochen im Bereich seiner seitlichen Begrenzungen (9, 10) führend in lotrechter Richtung nach oben. Der Sattel (1) ist über einen Zapfen (11) mit dem Mittelstück (3) verbunden, der sich im wesentlichen in lotrechter Richtung erstreckt und in einer sich im wesentlichen lotrechter Richtung erstreckenden und im Mittelstück (3) angeordneten Aufnahmebohrung (12) geführt ist. Im Bereich seiner dem Mittelstück (3) zugewandten unteren Begrenzung (13) weist der Sattel (1) eine den Zapfen (11) mindestens teilweise umschließende Sattelbohrung (14) auf. Es ist auch möglich, daß der Zapfen (11) mit dem Mittelstück (3) ein gemeinsames Teil bildet oder daß der Zapfen (11) mit Sattel (1) ein gemeinsames Teil bildet, das in der Aufnahmebohrung (12) geführt ist.

Zwischen dem Sattel (1) und dem Mittelstück (3) erstreckt sich ein die Länge der Sattelprothese anpassendes Distanzstück (15). Das Distanzstück (15) ist im wesentlichen als Distanzscheibe (16) ausgebildet, die eine im wesentlichen bezüglich ihres Mittelpunktes zentral angeordnete und in lotrechter Richtung verlaufende Führungsbohrung (17) aufweist. Durch die Führungsbohrung (17) hindurch erstreckt sich der Zapfen (11).

Es ist auch möglich, das Mittelstück (3) an die operative Situation anpaßbar auszubilden und beispielsweise in Richtung einer sich aus einer Gewichtskraft und einer Muskelkraft zusammensetzenden Kraftresultierenden mit konfigurierbarer Länge vorzusehen. Die Gewichtskraft verläuft im wesentlichen in lotrechter Richtung nach unten, die Muskelkraft weist in eine in einem Winkel zur Lotrechten verlaufende Richtung und wird von einem Muskel erzeugt, der einerseits im Bereich des Beckenknochens und andererseits im Bereich des Oberschenkelknochens (40) angewachsen ist.

Im Bereich der Aufnahmebohrung (12) erstreckt sich im wesentlichen in horizontaler Richtung eine Arretierungsbohrung (18), die ein Innengewinde (19) aufweist, das in ein Außengewinde (20) einer Sicherungsschraube (21) eingreift. Im Bereich ihres als Spitze (22) ausgebildeten und dem Zapfen (11) zugewandten Endes (23) beaufschlagt die Sicherungsschraube (21) bewegungshemmend den Zapfen (11).

Im Bereich der in lotrechter Richtung unteren Begrenzung (13) des Sattels (1) ist eine den Sattel (1) führende Lagerschale (24) angeordnet, die aus einem weicheren Material als der Sattel (1) ausgebildet ist. Die Lagerschale (24) ist aus Polyäthylen ausgebildet, es ist aber auch möglich, andere Kunststoffe oder sonstige zur Herstellung von Lagerschalen geeignete Materialien zu verwenden. Der Zapfen (11) ist in einer Lagerbuchse (25) geführt, die sich zwischen dem Zapfen (11) und dem Mittelstück (3) erstreckt.

Das Mittelstück (3) weist eine im wesentlichen in lotrechter Richtung verlaufende Längsschnittfläche (26) auf, die etwa s-förmig ausgebildet ist. Eine in lotrechter Richtung oben angeordnete Begrenzung (27) der Längsschnittfläche (26) verläuft etwa parallel zur unteren Begrenzung (13) des Sattel (1) und erstreckt sich im wesentlichen in horizontaler Richtung. Es ist aber auch möglich, die obere Begrenzung (27) gegenüber der Horizontalen geneigt anzuordnen. Eine in lotrechter Richtung untere Begrenzung (28) der Längsschnittfläche (26) weist einen Winkel zur Horizontalen von etwa 30 Grad auf. Es ist auch möglich, andere Neigungswinkel im Bereich von etwa 0 bis 70 Grad vorzusehen. Die Längsschnittfläche (26) weist darüber hinaus eine seitliche Begrenzung (29) auf, die ausgehend von der oberen Begrenzung (27) zunächst in Richtung einer in lotrechter Richtung ausgerichteten Sattelquerachse (30) verläuft und stetig in einen Auflagebreich (31) übergeht, der etwa einen Neigungswinkel von 16 Grad zur Horizontalen aufweist. Es ist aber auch möglich, einen Neigungswinkel im Bereich von 10 bis 30 Grad vorzusehen. Ausgehend vom Auflagebereich (31) verläuft die seitliche Begrenzung(29) sich stetig einer im wesentlichen in lotrechter Richtung ausgerichteten Schaftlängsachse (32) annähernd in Richtung auf die untere Begrenzung (28).

Im Bereich der unteren Begrenzung (28) weist das Mittelstück (3) eine Führungsbohrung (33) auf, die einen mit dem Schaft (2) verbundenen Führungszapfen (34) umschließt. Es ist aber auch möglich, daß die Führungsbohrung im Bereich des Schaftes (2) angeordnet und der Führungszapfen (34) mit dem Mittelstück (3) verbunden ist. Im Bereich der Führungsbohrung (33) ist ein den Führungszapfen (34) rotationshemmend beaufschlagendes Sicherungselement (37) angeordnet. Der Führungszapfen (34) ist im wesentlichen zylindrisch ausgebildet, es ist aber auch möglich, dem Führungszapfen (34) eine sich in Richtung auf den Boden (35) verjüngende konische Ausbildung zu verleihen. Der Schaft (2) weist ein ihn auf einem Oberschenkelknochen (40) abstützendes Auflageelement (41) auf. Das Auflageelement (41) weist eine im wesentlichen in lotrechter Richtung nach unten ausgerichtete Auflagefläche (42) auf, die mit einem Winkel von etwa 30 Grad zur Horizontalen ausgerichtet ist. Es ist aber auch möglich, daß die Auflagefläche (42) andere Winkel zur Horizontalen aufweist, die beispielsweise im Bereich von 0 bis 70 Grad liegen können.

Das Auflageelement (41) ist fest mit dem Schaft (2) verbunden und bildet mit diesem ein einheitliches Teil. Es ist aber auch möglich, das Auflageelement (41) als eigenständiges Teil auszubilden, das beispielsweise als mit dem Schaft (2) verschraubbares und im Bereich einer Nut (43) von diesem geführtes U-Profil ausgebildet sein kann. Der Schaft (2) ist im wesentlichen Bereich seiner Ausdehnung in einer im Oberschenkelknochen (40) vorgesehenen Knochenhöhle (47) angeordnet. Der Schaft (2) ist durch Knochenzement (48) mit dem Oberschenkelknochen (40) verbunden, es ist aber auch möglich, den Schaft (2) zementlos in den Oberschenkelknochen (40) einzusetzen.

Der Schaft (2) verläuft in lotrechter Richtung unterhalb des Auflageelementes (41) im wesentlichen in Richtung der Schaftlängsachse (32), es ist aber auch möglich, daß der Schaft (2) geschwungen ausgebildet ist. Es ist darüber hinaus möglich, daß der Schaft (2) als totale Femurprothese ausgebildet ist, der sich durch die Diaphyse in Richtung auf die Epiphyse erstreckt.

Im Bereich seiner äußeren Begrenzung ist der Schaft (2) im wesentlichen Bereich seiner Ausdehnung glattflächig ausgebildet, es ist aber auch möglich, daß in Richtung der Schaftlängsachse verlaufende Längsnuten (49) vorgesehen sind, die mit dem Schaft (2) in der Knochenhöhle (47) abstützenden Verzahnungen versehen sein können.

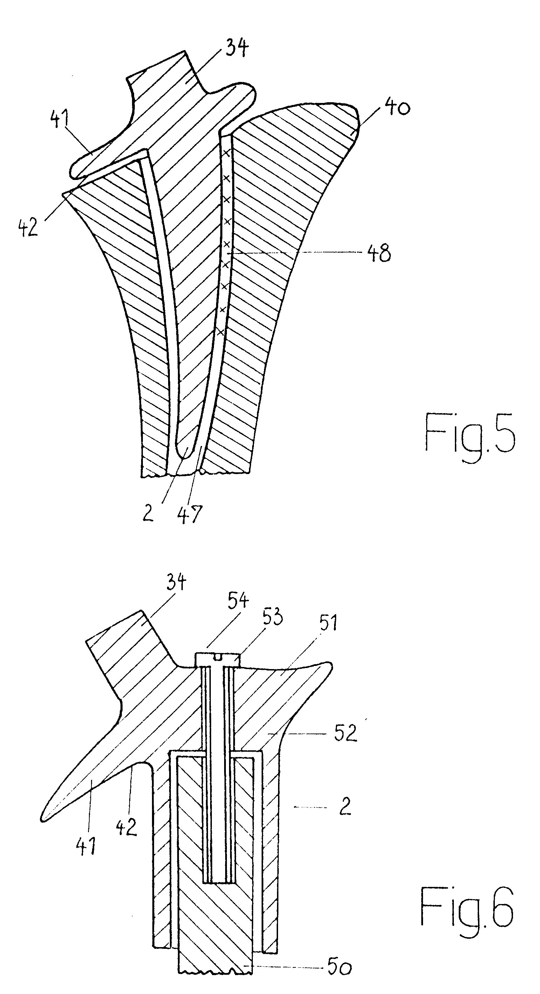

Bei der Ausführungsform des Schaftes gemäß Fig. 6 ist der Schaft (2) in ein Längselement (50) und einen Schaftkopf (51) unterteilt. Der Schaftkopf (51) ist als Reimersverschluß (52) ausgebildet, der das Längselement (50) im Bereich dessen in lotrechter Richtung oberen Endes teilweise umschließt und mit einem als Befestigungsschraube (53) ausgebildeten Halterungselement (54) mit diesem verbunden ist. Das Längselement (50) erstreckt sich im wesentlichen als zylindrischer Körper mit etwa konstanter Querschnittsfläche in Richtung der Schaftlängsachse (32), es ist aber auch möglich, daß das Längselement (50) ausgehend vom Schaftkopf (51) zunächst mit einem großen Querschnitt in Richtung der Schaftlängsachse (32) verläuft und sich ab einem Absatz (55) mit einem kleinen Querschnitt in Richtung der Schaftlängsachse erstreckt.

Im Bereich seiner dem Beckenknochen zugewandten Auflagefläche (77) weist der Sattel (1) eine reibungsvermindernde Beschichtung (78) auf, die als Keramikbeschichtung (79) ausgebildet ist.

Im Bereich des Mittelstückes (3) kann ein Stoßbelastungen aufnehmendes Dämpfungselement (80) angeordnet sein, das beispielsweise aus Silikonkunststoff ausgebildet ist.

Während einer Operation wird nach einer Entfernung der zerstörten Knochenteile zunächst der Schaft (2) in die Knochenhöhle (47) eingesetzt und im Beckenknochen die die Sattelmulde (7) aufnehmende Beckenmulde herausgearbeitet. Anschließend wird der Sattel (1) in die Beckenmulde hineingeschoben und ein geeignetes Distanzstück (15) zwischen dem Mittelstück (3) und dem Schaft (2) eingesetzt bzw. ein angepaßtes Mittelstück (3) eingesetzt. Die Sattelprothese ist dadurch in ihrer Dimensionierung paßgenau an die tatsächlichen Operationsverhältnisse angepaßt. Bei einer Ausführungsform nach Fig. 3 wird das Mittelstück (3) nach der Implantierung des Distanzstückes (15) durch den Zapfen (11) mit dem Sattel (1) verbunden. Abschließend wird der Zapfen (11) durch die Sicherungsschraube (21) verdrehsicher im Mittelstück (3) befestigt. Im Bereich des Schaftes (2) ist das Mittelstück (3) durch den Sicherungszapfen (36) verdrehsicher gehaltert. Nach einer Beendigung der Operation stützen sich die den Oberschenkelknochen (40) ausrichtenden Muskeln zum Teil auf dem Auflagebereich (31) ab und beaufschlagen den Oberschenkelknochen (40) über einen großen Hebelarm mit den von ihnen erzeugten Kräften. Die nach einer Beendigung der Operation und nach fortschreitender Genesung durch eine Bewegung des Beines auf die Prothese einwirkenden Momente verdrehen den Sattel (1) gegenüber dem Mittelstück (3) und entlasten die Beckenmulde von sie schädigenden Kräften. Der Sattel (1) befindet sich in Relation zum Beckenknochen im wesentlichen in Ruhe und stützt diesen großflächig ab. Aufgrund der im Bereich des Auflagebereichs (31) realisierten großen Hebelarme kann der Patient durch Muskelkontraktion gezielt seinen Bewegungsablauf beeinflussen und weitgehend einem natürlichen Bewegungsbild annähern.

## Patentansprüche

1. Sattelprothese zum Ersatz eines Hüftgelenkes mit zwei einen Sattel (1) zwischen sich ausbildenden Sattelhörnern (4,5), die mit einem in einen Oberschenkelknochen (40) einzusetzenden Schaft (2) verbunden sind, wobei eine sich im wesentlichen in Richtung einer aus einer Gewichtskraft und einer Muskelkraft ausgebildeten Kraftresultierenden erstreckende Sattelquerachse (30) versetzt zu einer sich im wesentlichen in lotrechter Richtung erstreckenden Schaftlängsachse (32) angeordnet ist, dadurch gekennzeichnet, daß zwischen dem Schaft (2) und dem Sattel (1) ein den Schaft (2) mit dem Sattel (1) verbindendes Mittelstück (3) angeordnet ist, mit dem der Sattel (1) um die quer zum Sattel (1) verlaufende Sattelquerachse (30) drehbar verbunden ist.

2. Sattelprothese nach Anspruch 1, dadurch gekennzeichnet, daß das Mittelstück (3) als ein separates Bauteil ausgebildet ist.

3. Sattelprothese nach Anspruch 1 und 2, dadurch gekennzeichnet, daß das Mittelstück (3) in einer in lotrechter Richtung verlaufenden Schnittebene eine etwa s-förmig ausgebildete Längsschnittfläche (26) aufweist.

4. Sattelprothese nach einem der Ansprüche 1 bis 3, dadurch gekennzeichnet, daß die Längsschnittfläche (26) im Bereich ihres dem Sattel (1) zugewandten Endes eine obere Begrenzung (27) aufweist, die einen Winkel zur Horizontalen begrenzt, der einen Wert im Bereich von 0 bis 30 Grad aufweist.

5. Sattelprothese nach einem der Ansprüche 1 bis 4, dadurch gekennzeichnet, daß die Längsschnittfläche (26) im Bereich ihres dem Schaft (2) zugewandten Endes eine in einem Winkel zur Horizontalen verlaufende untere Begrenzung (28) aufweist.

6. Sattelprothese nach Anspruch 5, dadurch gekennzeichnet, daß der Winkel einen Wertbereich von 0 bis 60 Grad aufweist.

7. Sattelprothese nach Anspruch 5 oder 6, dadurch gekennzeichnet, daß der Winkel einen Wert von etwa 30 Grad aufweist.

8. Sattelprothese nach mindestens einem der Ansprüche 1 bis 7, dadurch gekennzeichnet, daß der Sattel (1) im Bereich einer von den Sattelhörnern (4, 5) begrenzten Sattelmulde (7) eine breite, einen Beckenknochen (6) im Bereich einer Beckenmulde (8) halternde Auflagefläche aufweist.

9. Sattelprothese nach mindestens einem der Ansprüche 1 bis 8, dadurch gekennzeichnet, daß zwischen dem Sattel (1) und dem Mittelstück (3) mindestens ein Distanzstück (15) angeordnet ist.

10. Sattelprothese nach mindestens einem der Ansprüche 1 bis 9, dadurch gekennzeichnet, daß das Distanzstück (15) als Distanzscheibe (16) ausgebildet ist, die eine im wesentlichen in Richtung der Sattelquerachse (30) verlaufende Führungsbohrung (17) aufweist.

11. Sattelprothese nach mindestens einem der Ansprüche 1 bis 10. dadurch gekennzeichnet, daß das Mittelstück (3) mit dem Sattel (1) über einen Zapfen (11) verbunden ist.

12. Sattelprothese nach mindestens einem der Ansprüche 1 bis 11, dadurch gekennzeichnet, daß im Mittelstück (3) eine den Zapfen (11) führende und im wesentlichen in Richtung der Sattelquerachse (30) verlaufende Aufnahmebohrung (12) angeordnet ist.

13. Sattelprothese nach mindestens einem der Ansprüche 1 bis 12, dadurch gekennzeichnet, daß der Sattel (1) im Bereich seiner dem Mittelstück (3) zugewandten unteren Begrenzung (13) eine sich im wesentlichen in Richtung der Sattelquerachse (30) erstreckende und den Zapfen (11) im Bereich seines in lotrechter Richtung oberen Endes mindestens teilweise umschließende Sattelbohrung (14) aufweist.

14. Sattelprothese nach mindestens einem der Ansprüche 1 bis 13, dadurch gekennzeichnet, daß der Sattel (1) um die Sattelquerachse (30) drehbar mit dem Mittelstück (3) verbunden ist.

15. Sattelprothese nach mindestens einem der Ansprüche 1 bis 14, dadurch gekennzeichnet, daß im Bereich des Mittelstückes (3) ein den Zapfen (11) verdrehungshemmend beaufschlagendes Sperrelement angeordnet ist.

16. Sattelprothese nach Anspruch 15, dadurch gekennzeichnet, daß das Sperrelemement als Sicherungsschraube (21) ausgebildet ist, die mit einem Außengewinde (20) in ein Innengewinde (19) einer sie umschließenden Arretierungsbohrung eingreift und den Zapfen (11) mit einer diesem zugewandten Spitze (22) kraftschlüssig beaufschlagt.

17. Sattelprothese nach Anspruch 15 und 16, dadurch gekennzeichnet, daß sich die Arretierungsbohrung (18) im wesentlichen in horizontaler Richtung erstreckt.

18. Sattelprothese nach mindestens einem der Ansprüche 1 bis 14, dadurch gekennzeichnet, daß der Zapfen (11) fest mit dem Mittelstück (3) verbunden ist.

19. Sattelprothese nach mindestens einem der Ansprüche 1 bis 14, dadurch gekennzeichnet, daß der Zapfen (11) fest mit dem Sattel (1) verbunden und drehbar im Mittelstück (3) gelagert ist.

20. Sattelprothese nach mindestens einem der Ansprüche 1 bis 14, dadurch gekennzeichnet, daß der Zapfen (11) fest mit dem Distanzstück (15) verbunden ist.

21. Sattelprothese nach mindestens einem der Ansprüche 1 bis 20, dadurch gekennzeichnet, daß das Distanzstück (15) in Richtung der Sattelquerachse (30) eine an die jeweilige operative Situation anpaßbare Bemaßung aufweist.

22. Sattelprothese nach mindestens einem der Ansprüche 1 bis 21, dadurch gekennzeichnet, daß sich zwischen dem Sattel (1) und dem Mittelstück (3) mehrere einen sich zwischen dem Sattel (1) und dem Mittelstück (3) erstreckenden konfigurierbaren Abstand ausfüllende Distanzstücke (15) erstrecken.

23. Sattelprothese nach mindestens einem der Ansprüche 1 bis 22, dadurch gekennzeichnet, daß zwischen dem Sattel (1) und dem Mittelstück (3) eine den Sattel (1) verschleißarm führende Lagerschale (24) angeordnet ist.

24. Sattelprothese nach mindestens einem der Ansprüche 1 bis 23, dadurch gekennzeichnet, daß sich zwischen dem Zapfen (11) und dem Mittelstück (3) eine den Zapfen (11) führende Lagerbuchse (25) erstreckt.

25. Sattelprothese nach mindestens einem der Ansprüche 1 bis 24, dadurch gekennzeichnet, daß der Schaft (2) im Bereich seines dem Mittelstück (3) zugewandten Endes einen ihn mit dem Mittelstück (3) verbindenden Führungszapfen (34) aufweist.

26. Sattelprothese nach Anspruch 25, dadurch gekennzeichnet, daß der Führungszapfen (34) konisch ausgebildet ist.

27. Sattelprothese nach mindestens einem der Ansprüche 1 bis 26, dadurch gekennzeichnet, daß der Führungszapfen (34) in eine im Bereich des dem Schaft (2) zugewandten unteren Endes des Mittelstückes (3) angeordnete Führungsbohrung (33) eingreift, die sich im wesentlichen in Richtung einer Mittelachse 839) des Führungszapfens (34) erstreckt.

28. Sattelprothese nach mindestens einem der Ansprüche 1 bis 24, dadurch gekennzeichnet, daß der Führungszapfen (34) mit dem Mittelstück (3) verbunden ist und in eine im Bereich des Schaftes (2) vorgesehene Führungsbohrung (33) eingreift.

29. Sattelprothese nach mindestens einem der Ansprüche 1 bis 28, dadurch gekennzeichnet, daß der Führungszapfen (34) eine ihr rotationshemmend in der Führungsbohrung (33) halternde Drehsicherung aufweist.

30. Sattelprothese nach mindestens einem der Ansprüche 1 bis 29, dadurch gekennzeichnet, daß sich der als Konus ausgebildete Führungszapfen (34) in Richtung auf das Mittelstück (3) verjüngt.

31. Sattelprothese nach mindestens einem der Ansprüche 1 bis 25, dadurch gekennzeichnet, daß der Führungszapfen (34) im wesentlichen zylindrisch ausgebildet ist.

32. Sattelprothese nach mindestens einem der Ansprüche 1 bis 25, dadurch gekennzeichnet, daß der Führungszapfen (34) bezüglich seiner Mittelachse (39) asymmetrisch ausgebildet ist.

33. Sattelprothese nach mindestens einem der Ansprüche 1 bis 32, dadurch gekennzeichnet, daß der Schaft (2) ein Auflageelement (41) aufweist, das mit einer einem Oberschenkelknochen (40) zugewandten Auflagefläche (42) auf einer der Auflagefläche (42) zugewandten oberen Begrenzung des Oberschenkelknochens (40) aufliegt.

34. Sattelprothese nach mindestens einem der Ansprüche 1 bis 33, dadurch gekennzeichnet, daß der Schaft (2) in einem wesentlichen Bereich seiner Ausdehnung glattflächig ausgebildet ist.

35. Sattelprothese nach mindestens einem der Ansprüche 1 bis 33, dadurch gekennzeichnet, daß der Schaft (2) mindestens in einem Bereich seiner Ausdehnung ihn im Oberschenkelknochen halternde Nuten (49) aufweist.

36. Sattelprothese nach Anspruch 35, dadurch gekennzeichnet, daß die Nuten als Längsnuten (49) mit scharfen schneidenden Kanten ausgebildet sind.

37. Sattelprothese nach mindestens einem der Ansprüche 1 bis 36, dadurch gekennzeichnet, daß der Schaft (2) zementlos in einer ihn aufnehmenden im Oberschenkelknochen (40) angeordneten Knochenhohle (47) befestigt ist.

38. Sattelprothese nach mindestens einem der Ansprüche 1 bis 37, dadurch gekennzeichnet, daß der Schaft (2) lang ausgebildet und im Bereich seines in lotrechter Richtung unteren Endes mit dem Oberschenkelknochen (40) verbunden ist.

39. Sattelprothese nach Anspruch 37 oder 38, dadurch gekennzeichnet, daß der Schaft (2) im Bereich seines in lotrechter Richtung oberen Endes von in die Knochenhohle (47) gestopftem Knochenmaterial umgeben ist.

40. Sattelprothese nach mindestens einem der Ansprüche 1 bis 39, dadurch gekennzeichnet, daß der Schaft (2) mindestens bereichsweise von ihn in der Knochenhöhle (47) halterndem Knochenzement (48) umgeben ist.

41. Sattelprothese nach Anspruch 1 bis 40, dadurch gekennzeichnet, daß der Schaft (2) als totale Femurprothese ausgebildet ist.

42. Sattelprothese nach mindestens einem der Ansprüche 1 bis 41, dadurch gekennzeichnet, daß der Schaft (2) in ein Längselement (50) und ein das Längselement (50) im Bereich seines in lotrechter Richtung oberen Endes mindestens teilweise umschließenden Schaftkopfes (51) unterteilt ist.

43. Sattelprothese nach Anspruch 42, dadurch gekennzeichnet, daß der Schaftkopf (51) als Reimersverschluß (52) ausgebildet ist.

44. Sattelprothese nach Anspruch 42 oder 43, dadurch gekennzeichnet, daß der Schaftkopf (51) eine ihn mit dem Längselement (50) verbindende Sicherung aufweist.

45. Sattelprothese nach Anspruch 44, dadurch gekennzeichnet, daß die Sicherung als Befestigungsschraube (53) ausgebildet ist.

46. Sattelprothese nach Anspruch 44, dadurch gekennzeichnet, daß die Sicherung als Blockierstift ausgebildet ist.

47. Sattelprothese nach mindestens einem der Ansprüche 1 bis 46, dadurch gekennzeichnet, daß der Sattel (1) im wesentlichen aus Metall ausgebildet ist.

48. Sattelprothese nach Anspruch 47, dadurch gekennzeichnet, daß der Sattel (1) im wesentlichen aus Titan ausgebildet ist.

49. Sattelprothese nach mindestens einem der Ansprüche 1 bis 46, dadurch gekennzeichnet, daß der Sattel (1) im wesentlichen aus Kunststoff ausgebildet ist.

50. Sattelprothese nach mindestens einem der Ansprüche 1 bis 46, dadurch gekennzeichnet, daß der Sattel (1) im wesentlichen aus Keramik ausgebildet ist.

51. Sattelprothese nach mindestens einem der Ansprüche 1 bis 46, dadurch gekennzeichnet, daß der Sattel (1) mindesten im Bereich seiner dem Beckenknochen (6) zugewandten Auflagefläche eine reibungsmindernde Beschichtung (78) aufweist.

52. Sattelprothese nach Anspruch 51, dadurch gekennzeichnet, daß die Beschichtung (78) als Keramikbeschichtung (79) ausgebildet ist.

53. Sattelprothese nach Anspruch 51 oder 52, dadurch gekennzeichnet, daß die Beschichtung (78) im wesentlichen die Form eines Sattels aufweist.

54. Sattelprothese nach mindestens einem der Ansprüche 1 bis 53, dadurch gekennzeichnet, daß das Mittelstück (3) im wesentlichen aus Metall ausgebildet ist.

55. Sattelprothese nach Anspruch 54, dadurch gekennzeichnet, daß das Mittelstück (3) im wesentlichen aus Titan ausgebildet ist.

56. Sattelprothese nach mindestens einem der Ansprüche 1 bis 53, dadurch gekennzeichnet, daß das Mittelstück (3) im wesentlichen aus Kunststoff ausgebildet ist.

57. Sattelprothese nach mindestens einem der Ansprüche 1 bis 53, dadurch gekennzeichnet, daß das Mittelstück (3) im wesentlichen aus Keramik ausgebildet ist.

58. Sattelprothese nach mindestens einem der Ansprüche 1 bis 57, dadurch gekennzeichnet, daß die Lagerschale (24) aus Kunststoff ausgebildet ist.

59. Sattelprothese nach Anspruch 58, dadurch gekennzeichnet, daß die Lagerschale (24) aus Polyäthylen ausgebildet ist.

60. Sattelprothese nach mindestens einem der Ansprüche 1 bis 59, dadurch gekennzeichnet, daß die Lagerbuchse (25) aus Kunststoff ausgebildet ist.

61. Sattelprothese nach mindestens einem der Ansprüche 1 bis 60, dadurch gekennzeichnet, daß das Auflageelement (41) als den Schaft (2) großflächig auf dem Oberschenkelknochen (40) abstützender Kragen ausgebildet ist.

62. Sattelprothese nach mindestens einem der Ansprüche 1 bis 61, dadurch gekennzeichnet, daß der Zapfen (11) im Bereich seines in lotrechter Richtung unteren Endes eine ihn an ein Positionierungswerkzeug (64) anschließende Kupplung (63) aufweist.

63. Sattelprothese nach mindestens einem der Ansprüche 1 bis 62, dadurch gekennzeichnet, daß die Sattelquerachse (30) in Richtung einer die Sattelmulde (7) im Bereich ihrer Verbindung zur Beckenmulde (8) beaufschlagenden und in Richtung der Kraftresultierenden wirkenden Druckkraft verläuft.

64. Sattelprothese nach mindestens einem der Ansprüche 1 bis 63, dadurch gekennzeichnet, daß das Mittelstück (3) als anpaßbares Element ausgebildet ist, das eine in Richtung einer Zapfenmittelachse (81) des Zapfens (11) konfigurierbare Ausdehnung aufweist.

65. Sattelprothese nach mindestens einem der Ansprüche 1 bis63, dadurch gekennzeichnet, daß die Zapfenmittelachse (81) sich im wesentlichen in Richtung einer Kraftresultierenden erstreckt, die aus der Gewichtskraft und der Muskelkraft zusammengesetzt ist.

66. Sattelprothese nach Anspruch 65, dadurch gekennzeichnet, daß die Zapfenmittelachse (81) einen Winkel zur Lotrechten von etwa 0 bis 30 Grad aufweist.

67. Sattelprothese nach Anspruch 66, dadurch gekennzeichnet, daß der Winkel einen Wert von etwa 16 Grad aufweist.

68. Sattelprothese nach mindestens einem der Ansprüche 1 bis 67, dadurch gekennzeichnet, daß sich der Führungszapfen (34) im Bereich seines in lotrechter Richtung oberen Endes durch eine Ebene hindurch erstreckt, die von der dem Sattel (1) zugewandten oberen Begrenzung des Mittelstückes (3) aufgespannt ist.

69. Sattelprothese nach mindestens einem der Ansprüche 1 bis 68, dadurch gekennzeichnet, daß im Bereich des Mittelstückes (3) mindestens ein Stoßbelastungen aufnehmendes Dämpfungselement (80) angeordnet ist.

70. Sattelprothese nach mindestens einem der Ansprüche 1 bis 69, dadurch gekennzeichnet, daß eine von der dem Sattel (1) zugewandten oberen Begrenzung (27) des Mittelstückes (3) aufgespannte Drehebene im wesentlichen senkrecht zur Kraftresultierenden verläuft.

71. Sattelprothese nach mindestens einem der Ansprüche 1 bis 70, dadurch gekennzeichnet, daß die s-förmige Längsschnittfläche (26) einen sich im wesentlichen linear erstreckenden Mittelbereich aufweist, dessen Länge und Neigung zur Horizontalen ein fest vorgegebenes konstantes Verhältnis aufweist.

## Claims

1. A saddle prosthesis for replacement of a hip joint comprising two saddle horns (4, 5) which form a saddle (1) between them and which are connected to a shank (2) to be inserted into a four (40), wherein a saddle transverse axis (30) extending substantially in the direction of a resultant force formed from a force due to weight and a muscle force is arranged in displaced relationship relative to a shank longitudinal axis (32) extending substantially in a perpendicular direction, characterised in that disposed between the shank (2) and the saddle (1) is a central portion (3) which connects the shank (2) to the saddle (1) and to which the saddle (1) is connected rotatably about the saddle transverse axis (30) which extends transversely relative to the saddle (1).

2. A saddle prosthesis according to claim 1 characterised in that the central portion (3) is in the form of a separate component.

3. A saddle prosthesis according to claim 1 and claim 2 characterised in that in a section plane extending in a perpendicular direction the central portion (3) has a longitudinal section surface (26) which is of a substantially s-shaped configuration.

4. A saddle prosthesis according to one of claims 1 to 3 characterised in that the longitudinal section surface (26), in the region of its end towards the saddle (1), has an upper boundary (27) defining relative to the horizontal an angle which is of a value in the range of from 0 to 30°.

5. A saddle prosthesis according to one of claims 1 to 4 characterised in that the longitudinal section surface (26), in the region of its end towards the shank (2), has a lower boundary (28) extending at an angle relative to the horizontal.

6. A saddle prosthesis according to claim 5 characterised in that the angle has a range of values of from 0 to 60°.

7. A saddle prosthesis according to claim 5 or claim 6 characterised in that the angle is of a value of about 30°.

8. A saddle prosthesis according to at least one of claims 1 to 7 characterised in that, in the region of a saddle hollow (7) which is defined by the saddle horns (4, 5), the saddle (1) has a wide support surface which aunts a hip bone (6) in the region of a pelvic hollow (8).

9. A saddle prosthesis according to at least one of claims 1 to 8 characterised in that at least one spacer portion (15) is arranged between the saddle (1) and the central portion (3).

10. A saddle prosthesis according to at least one of claims 1 to 9 characterised in that the spacer portion (15) is in the form of a spacer disc (16) which has a guide bore (17) extending substantially in the direction of the saddle transverse axis (30).

11. A saddle prosthesis according to at least one of claims 1 to 10 characterised in that the central portion (3) is connected to the saddle (1) by way of a pin (11).

12. A saddle prosthesis according to at least one of claims 1 to 11 characterised in that disposed in the central portion (3) is a receiving bore (12) which guides the pin (11) and which extends substantially in the direction of the saddle transverse axis (30).

13. A saddle prosthesis according to at least one of claims 1 to 12 characterised in that in the region of its lower boundary (13) which is towards the central portion (3), the saddle (1) has a saddle bore (14) which extends substantially in the direction of the saddle transverse axis (30) and which at least partially surrounds the pin (11) in the region of its end which is the upper end in the perpendicular direction.

14. A saddle prosthesis according to at least one of claims 1 to 13 characterised in that the saddle (1) is connected to the central portion (3) rotatably about the saddle transverse axis (30).

15. A saddle prosthesis according to at least one of claims 1 to 14 characterised in that provided in the region of the central portion (3) is a locking element which acts on the pin (11) to check twisting.

16. A saddle prosthesis according to claim 15 characterised in that the locking element is in the form of a securing screw (21) which engages with an external screwthread (20) into an internal screwthread (19) in an arresting bore enclosing same, and force-lockingly acts on the pin (11) with a tip (22) which is towards same.

17. A saddle prosthesis according to claim 15 and claim 16 characterised in that the arresting bore (18) extends substantially in a horizontal direction.

18. A saddle prosthesis according to at least one of claims 1 to 14 characterised in that the pin (11) is fixedly connected to the central portion (3).

19. A saddle prosthesis according to at least one of claims 1 to 14 characterised in that the pin (11) is fixedly connected to the saddle (1) and is mounted rotatably in the central portion (3).

20. A saddle prosthesis according to at least one of claims 1 to 14 characterised in that the pin (11) is fixedly connected to the spacer portion (15).

21. A saddle prosthesis according to at least one of claims 1 to 20 characterised in that the spacer portion (15) is of a dimension, in the direction of the saddle transverse axis (13), which can be adapted to the respective operative situation.

22. A saddle prosthesis according to at least one of claims 1 to 21 characterised in that extending between the saddle (1) and the central portion (3) are a plurality of spacer portions (15) which fill a configuratable spacing extending between the saddle (1) and the central portion (3).

23. A saddle prosthesis according to at least one of claims 1 to 22 characterised in that arranged between the saddle (1) and the central portion (3) is a bearing shell (24) for guiding the saddle (1) with a low rate of wear.

24. A saddle prosthesis according to at least one of claims 1 to 23 characterised in that a bearing bush (25) for guiding the pin (11) extends between the pin (11) and the central portion (3).

25. A saddle prosthesis according to at least one of claims 1 to 24 characterised in that, in the region of its end towards the central portion (3), the shank (2) has a guide pin (34) which connects it to the central portion (3).

26. A saddle prosthesis according to claim 25 characterised in that the guide pin (34) is of a conical configuration.

27. A saddle prosthesis according to at least one of claims 1 to 26 characterised in that the guide pin (34) engages into a guide bore (33) which is disposed in the region of the lower end of the central portion (3) which is towards the shank (2), the guide bore extending substantially in the direction of a central axis (39) of the guide pin (34).

28. A saddle prosthesis according to at least one of claims 1 to 24 characterised in that the guide pin (34) is connected to the central portion (3) and engages into a guide bore (33) in the region of the shank (2).

29. A saddle prosthesis according to at least one of claims 1 to 28 characterised in that the guide pin (34) has a rotation-securing bans for holding it in the guide bore (33) with a rotation-checking action.

30. A saddle prosthesis according to at least one of claims 1 to 29 characterised in that the guide pin (34) which is of a conical configuration tapers towards the central portion (3).

31. A saddle prosthesis according to at least one of claims 1 to 25 characterised in that the guide pin (34) is of a substantially cylindrical configuration.

32. A saddle prosthesis according to at least one of claims 1 to 25 characterised in that the guide pin (34) is of an asymmetrical configuration relative to its central axis (39).

33. A saddle prosthesis according to at least one of claims 1 to 32 characterised in that the shank (2) has a support element (41) which, with a support surface (42) which is towards a femur (40), lies on an upper boundary of the femur (40), which is towards the support surface (42).

34. A saddle prosthesis according to at least one of claims 1 to 33 characterised in that the shank (2) is of a smooth-surface configuration in a substantial region of its extent.

35. A saddle prosthesis according to at least one of claims 1 to 33 characterised in that at least in a region of its extent the shank (2) has grooves (49) for holding it in the femur.

36. A saddle prosthesis according to claim 35 characterised in that the grooves are in the form of longitindinal grooves (49) with sharp cutting edges.

37. A saddle prosthesis according to at least one of claims 1 to 36 characterised in that the shank (2) is cementlessly fixed in a bone cavity (47) for accommodating it in the femur (40).

38. A saddle prosthesis according to at least one of claims 1 to 37 characterised in that the shank (2) is long and in the region of its end which is the lower end in a perpendicular direction is connected to the femur (40).

39. A saddle prosthesis according to claim 37 or claim 38 characterised in that, in the region of its end which is the upper end in a perpendicular direction, the shank (2) is surrounded by bone material which is tamped into the bone cavity (47).

40. A saddle prosthesis according to at least one of claims 1 to 39 characterised in that the shank (2) is surrounded at least in a region-wise manner by bone cement (48) for holding it in the bone cavity (47).

41. A saddle prosthesis according to claims 1 to 40 characterised in that the shank (2) is in the form of a total femur prosthesis.

42. A saddle prosthesis according to at least one of claims 1 to 41 characterised in that the shank (2) is subdivided into a longitudinal element (50) and a shank head (51) which at least partially encloses the longitudinal element (50) in the region of its end which is the upper end in the perpendicular direction.

43. A saddle prosthesis according to claim 42 characterised in that the shank head (51) is in the form of a Reimer fastening means (52).

44. A saddle prosthesis according to claim 42 or claim 43 characterised in that the shank head (51) has a securing means connecting it to the longitudinal element (50).

45. A saddle prosthesis according to claim 44 characterised in that the securing means is in the form of a fixing screw (53).

46. A saddle prosthesis according to claim 44 characterised in that the securing means is in the form of a blocking pin.

47. A saddle prosthesis according to at least one of claims 1 to 46 characterised in that the saddle (1) is made substantially from metal.

48. A saddle prosthesis according to claim 47 characterised in that the saddle (1) is made substantially from titanium.

49. A saddle prosthesis according to at least one of claims 1 to 46 characterised in that the saddle (1) is made substantially from plastics material.

50. A saddle prosthesis according to at least one of claims 1 to 46 characterised in that the saddle (1) is made substantially from ceramic.

51. A saddle prosthesis according to at least one of claims 1 to 46 characterised in that the saddle (1) has a friction-reducing coating (78) at least in the region of its support surface which is towards the hip bone (6).

52. A saddle prosthesis according to claim 51 characterised in that the coating (78) is in the form of a ceramic coating (79).

53. A saddle prosthesis according to claim 51 or claim 52 characterised in that the coating (78) is substantially in the shape of a saddle.

54. A saddle prosthesis according to at least one of claims 1 to 53 characterised in that the central portion (3) is made substantially from metal.

55. A saddle prosthesis according to claim 54 characterised in that the central portion (3) is made substantially from titanium.

56. A saddle prosthesis according to at least one of claims 1 to 53 characterised in that the central portion (3) is made substantially from plastics material.

57. A saddle prosthesis according to at least one of claims 1 to 53 characterised in that the central portion (3) is made substantially from ceramic.

58. A saddle prosthesis according to at least one of claims 1 to 57 characterised in that the bearing shell (24) is made from plastics material.

59. A saddle prosthesis according to claim 58 characterised in that the bearing shell (24) is made from polyethylene.

60. A saddle prosthesis according to at least one of claims 1 to 59 characterised in that the bearing bush (25) is made from plastics material.

61. A saddle prosthesis according to at least one of claims 1 to 60 characterised in that the support element (41) is in the form of a collar for supporting the shank (2) over a large area on the femur (40).

62. A saddle prosthesis according to at least one of claims 1 to 61 characterised in that, in the region of its end which is the lower end in the perpendicular direction, the pin (11) has a coupling (63) connecting it to a positioning tool (64).

63. A saddle prosthesis according to at least one of claims 1 to 62 characterised in that the saddle transverse axis (30) is in the direction of a pressure force which acts on the saddle hollow (7) in the region of its connection to the pelvic hollow (8) and which acts in the direction of the resultant force.

64. A saddle prosthesis according to at least one of claims 1 to 63 characterised in that the central portion (3) is in the form of a matchable element which is of an extent which is configuratable in the direction of a central axis (81) of the pin (11).

65. A saddle prosthesis according to at least one of claims 1 to 63 characterised in that the central axis (81) of the pin extends substantially in the direction of a resultant force which is composed of the force due to weight and muscle force.

66. A saddle prosthesis according to claim 65 characterised in that the pin central axis (81) is at an angle of between about 0 and 30° relative to the perpendicular.

67. A saddle prosthesis according to claim 66 characterised in that the angle is of a value of about 16°.

68. A saddle prosthesis according to at least one of claims 1 to 67 characterised in that the guide pin (34), in the region of its end which is the upper end in the perpendicular direction, extends through a plane which is defined by the upper boundary of the central portion (3), which is towards the saddle (1).

69. A saddle prosthesis according to at least one of claims 1 to 68 characterised in that disposed in the region of the central portion (3) is at least one damping element (80) for absorbing shock loadings.

70. A saddle prosthesis according to at least one of claims 1 to 69 characterised in that a rotational plane defined by the upper boundary (27) of the central portion (3), which is towards the saddle (1), extends substantially perpendicularly to the resultant force.

71. A saddle prosthesis according to at least one of claims 1 to 70 characterised in that the s-shaped longitudinal section surface (26) has a substantially linearly extending central region whose length and inclination relative to the horizontal has a fixedly predetermined constant relationship.

## Revendications

1. Prothèse en selle pour le remplacement d'une articulation de hanche avec deux cornes (4, 5) formant entre elles une selle (1) et reliées avec un corps (2) devant être introduit dans un fémur (40), dans laquelle un axe transversal de selle (30) s'étendant sensiblement dans la direction d'une résultante de force formée par un poids et une force musculaire est décalé par rapport à un axe longitudinal de corps (32) sensiblement vertical, caractérisée en ce qu'une pièce intermédiaire (3) reliant le corps (2) et la selle (1) est disposée entre le corps (2) et la selle (1), à laquelle la selle (1) est reliée avec possibilité de rotation autour de l'axe transversal de selle (30) perpendiculaire à la selle (1).

2. Prothèse en selle selon la revendication 1, caractérisée en ce que la pièce intermédiaire (3) est conçue comme un élément séparé.

3. Prothèse en selle selon l'une ou l'ensemble des revendications 1 et 2, caractérisée en ce que la pièce intermédiaire (3) présente une surface de coupe longitudinale (26) approximativement en forme de S dans un plan de coupe orienté verticalement.

4. Prothèse en selle selon l'une ou l'ensemble des revendications 1 à 3, caractérisée en ce que la surface de coupe longitudinale (26) présente dans la zone de son extrémité orientée vers la selle (1) une délimitation supérieure (27) qui délimite un angle par rapport à l'horizontale d'une valeur allant de 0 à 30 degrés.

5. Prothèse en selle selon l'une ou l'ensemble des revendications 1 à 4, caractérisée en ce que la surface de coupe longitudinale (26) présente dans la zone de son extrémité orientée vers le corps (2) une délimitation inférieure (28) orientée à un certain angle de l'horizontale.

6. Prothèse en selle selon la revendication 5, caractérisée en ce que l'angle présente une plage de valeurs de 0 à 60 degrés.

7. Prothèse en selle selon la revendication 5 ou 6, caractérisée en ce que l'angle présente une valeur de 30 degrés environ.

8. Prothèse en selle selon l'une ou l'ensemble des revendications 1 à 7, caractérisée en ce que la selle (1) présente dans la zone d'une cuvette de selle (7) délimitée par les cornes de selle (4, 5) une surface d'appui large et maintenant un os du bassin (6) dans la zone d'un cotyle (8).

9. Prothèse en selle selon l'une ou l'ensemble des revendications 1 à 8, caractérisée en ce qu'une pièce d'écartement (15) au moins est prévue entre la selle (1) et la pièce intermédiaire (3).

10. Prothèse en selle selon l'une ou l'ensemble des revendications 1 à 9, caractérisée en ce que la pièce d'écartement (15) est conçue comme une rondelle d'écartement (16), qui présente un trou de guidage (17) orienté sensiblement dans la direction de l'axe transversal de la selle (30).

11. Prothèse en selle selon l'une ou l'ensemble des revendications 1 à 10, caractérisée en ce que la pièce intermédiaire (3) est reliée à la selle par une cheville (11).

12. Prothèse en selle selon l'une ou l'ensemble des revendications 1 à 11, caractérisée en ce qu'un trou de réception (12) guidant la cheville (11) et orienté sensiblement dans la direction de l'axe transversal de la selle (30) est prévu dans la pièce intermédiaire (3).

13. Prothèse en selle selon l'une ou l'ensemble des revendications 1 à 12, caractérisée en ce que la selle (1) présente dans la zone de sa délimitation inférieure (13) orientée vers la pièce intermédiaire (3) un trou de selle (14) s'étendant sensiblement dans la direction de l'axe transversal de la selle (30) et entornrant au moins partiellement la cheville (11) dans la zone de son extrémité supérieure dans le sens vertical.

14. Prothèse en selle selon l'une ou l'ensemble des revendications 1 à 13, caractérisée en ce que la selle (1) et l'axe transversal de la selle (30) sont reliés à la pièce intermédiaire (3) avec possibilité de rotation.

15. Prothèse en selle selon l'une ou l'ensemble des revendications 1 à 14, caractérisée en ce qu'un élément de verrouillage sollicitant la cheville (11) en vue d'empêcher sa rotation est disposé dans la zone de la pièce intermédiaire (3).

16. Prothèse en selle selon la revendication 15, caractérisée en ce que l'élément de verrouillage est conçu comme une vis de sécurité (21), qui se met en prise par un filet extérieur (20) dans un filet intérieur (19) d'un trou d'arrêt qui l'entoure et sollicite la goupille (11) par adhérence avec une pointe (22) orientée vers celle-ci.

17. Prothèse en selle selon les revendications 15 et 16, caractérisée en ce que le trou d'arrêt (18) s'étend sensiblement à l'horizontale.

18. Prothèse en selle selon l'une ou l'ensemble des revendications 1 à 14, caractérisée en ce que la cheville (11) est fixée à la pièce intermédiaire (3).

19. Prothèse en selle selon l'une ou l'ensemble des revendications 1 à 14, caractérisée en ce que la cheville (11) est reliée de manière fixe à la selle (1) et supportée avec possibilité de rotation dans la pièce intermédiaire (3).

20. Prothèse en selle selon l'une ou l'ensemble des revendications 1 à 14, caractérisée en ce que la cheville (11) est reliée de manière fixe à la pièce d'écartement (15).

21. Prothèse en selle selon l'une ou l'ensemble des revendications 1 à 20, caractérisée en ce que la pièce d'écartement (15) présente dans la direction de l'axe transversal de la selle (30) une dimension adaptable à chaque situation opératoire.

22. Prothèse en selle selon l'une ou l'ensemble des revendications 1 à 21, caractérisée en ce que plusieurs pièces d'écartement (15) comblant un écart pouvant être configuré entre la selle (1) et la pièce intermédiaire (3) s'étendent entre la selle (1) et la pièce intermédiaire (3).

23. Prothèse en selle selon l'une ou l'ensemble des revendications 1 à 22, caractérisée en ce qu'une cuvette de support (24) guidant la selle (1) avec une faible usure est disposée entre la selle (1) et la pièce intermédiaire (3).

24. Prothèse en selle selon l'une ou l'ensemble des revendications 1 à 23, caractérisée en ce qu'un coussinet (25) guidant la cheville (11) s'étend entre la cheville (11) et la pièce intermédiaire (3).

25. Prothèse en selle selon l'une ou l'ensemble des revendications 1 à 24, caractérisée en ce que le corps (2) présente dans la zone de son extrémité orientée vers la pièce intermédiaire (3) une goupille de guidage (34) qui le relie à la pièce intermédiaire (3).

26. Prothèse en selle selon la revendication 25, caractérisée en ce que la goupille de guidage (34) est de forme conique.

27. Prothèse en selle selon l'une ou l'ensemble des revendications 1 à 26, caractérisée en ce que la goupille de guidage (34) se met en prise dans un trou de guidage (33) disposé dans la zone de l'extrémité inférieure de la pièce intermédiaire (3) orientée vers la tige (2), lequel s'étend sensiblement dans la direction d'un axe médian (39) de la goupille de guidage (34).

28. Prothèse en selle selon l'une ou l'ensemble des revendications 1 à 24, caractérisée en ce que la goupille de guidage (34) est reliée à la pièce intermédiaire (3) et se met en prise dans un trou de guidage (33) prévu dans la zone du corps (2).

29. Prothèse en selle selon l'une ou l'ensemble des revendications 1 à 28, caractérisée en ce que la goupille de guidage (34) présente une sécurité en rotation qui la maintient en empêchant sa rotation dans le trou de guidage (33).

30. Prothèse en selle selon l'une ou l'ensemble des revendications 1 à 29, caractérisée en ce que la goupille de guidage (34) conçue comme un cône se rétrécit en direction de la pièce intermédiaire (3).

31. Prothèse en selle selon l'une ou l'ensemble des revendications 1 à 25, caractérisée en ce que la goupille de guidage (34) est de forme sensiblement cylindrique.

32. Prothèse en selle selon l'une ou l'ensemble des revendications 1 à 25, caractérisée en ce que la goupille de guidage (34) est de forme asymétrique par rapport à son axe médian (39).

33. Prothèse en selle selon l'une ou l'ensemble des revendications 1 à 32, caractérisée en ce que le corps (2) présente un élément d'appui (41), qui repose par une surface d'appui (42) orientée vers un fémur (40) sur une délimitation supérieure du fémur (40) orientée vers la surface d'appui (42).

34. Prothèse en selle selon l'une ou l'ensemble des revendications 1 à 33, caractérisée en ce que le corps (2) est conformé dans un secteur essentiel de son étendue avec une surface lisse.

35. Prothèse en selle selon l'une ou l'ensemble des revendications 1 à 33, caractérisée en ce que la tige (2) présente au moins dans un secteur de son étendue des rainures (49) qui le maintiennent dans le fémur.

36. Prothèse en selle selon la revendication 35, caractérisée en ce que les rainures sont conçues comme des rainures longitudinales (49) avec des bords aigus et coupants.

37. Prothèse en selle selon l'une ou l'ensemble des revendications 1 à 36, caractérisée en ce que le corps (2) est fixé sans ciment dans un trou de l'os (47) disposé dans le fémur (40) et qui le reçoit.

38. Prothèse en selle selon l'une ou l'ensemble des revendications 1 à 37, caractérisée en ce que le corps (2) est de forme allongée et relié avec le fémur (40) dans la zone de son extrémité inférieure dans le sens vertical.

39. Prothèse en selle selon la revendication 37 ou 38, caractérisée en ce que le corps (2) est entouré dans la zone de son extrémité supérieure dans le sens vertical par du matériau osseux bourré dans le trou de l'os (47).

40. Prothèse en selle selon l'une ou l'ensemble des revendications 1 à 39, caractérisée en ce que le corps (2) est entouré au moins par secteurs de ciment osseux (48) le maintenant dans le trou de l'os (47).

41. Prothèse en selle selon l'une ou l'ensemble des revendications 1 à 40, caractérisée en ce que le corps (2) est conçu comme une prothèse fémorale totale.

42. Prothèse en selle selon l'une ou l'ensemble des revendications 1 à 41, caractérisée en ce que le corps (2) est divisé en un élément longitudinal (50) et une tête de corps (51) entourant au moins partiellement l'élément longitudinal (50) dans la zone de son extrémité supérieure dans le sens vertical.

43. Prothèse en selle selon la revendication 42, caractérisée en ce que la tête du corps (51) est conçue comme une fermeture Reimer (52).

44. Prothèse en selle selon la revendication 42 ou 43, caractérisée en ce que la tête du corps (51) présente une sécurité la reliant à l'élément longitudinal (50).

45. Prothèse en selle selon la revendication 44, caractérisée en ce que la sécurité est conçue comme une vis de fixation (53).

46. Prothèse en selle selon la revendication 44, caractérisée en ce que la sécurité est conçue comme une goupille de blocage.

47. Prothèse en selle selon l'une ou l'ensemble des revendications 1 à 46, caractérisée en ce que la sel le (1) est essentiellement composée de métal.

48. Prothèse en selle selon la revendication 47, caractérisée en ce que la selle (1) est essentiellement composée de titane.

49. Prothèse en selle selon l'une ou l'ensemble des revendications 1 à 46, caractérisée en ce que la selle (1) est essentiellement composée de plastique.

50. Prothèse en selle selon l'une ou l'ensemble des revendications 1 à 46, caractérisée en ce que la selle (1) est essentiellement composée de céramique.

51. Prothèse en selle selon l'une ou l'ensemble des revendications 1 à 46, caractérisée en ce que la selle (1) présente au moins dans la zone de sa surface d'appui orientée vers l'os du bassin (6) un revêtement (78) réduisant le frottement.

52. Prothèse en selle selon la revendication 51, caractérisée en ce que le revêtement (78) est conçu comme un revêtement de céramique (79).

53. Prothèse en selle selon la revendication 51 ou 52, caractérisée en ce que le revêtement (78) présente sensiblement la forme d'une selle.

54. Prothèse en selle selon l'une ou l'ensemble des revendications 1 à 53, caractérisée en ce que la pièce intermédiaire (3) est essentiellement composée de métal.

55. Prothèse en selle selon la revendication 54, caractérisée en ce que la pièce intermédiaire (3) est essentiellement composée de titane.

56. Prothèse en selle selon l'une ou l'ensemble des revendications 1 à 53, caractérisée en ce que la pièce intermédiaire (3) est essentiellement composée de plastique.

57. Prothèse en selle selon selon l'une ou l'ensemble des revendications 1 à 53, caractérisée en ce que la pièce intermédiaire (3) est essentiellement composée de céramique.

58. Prothèse en selle selon l'une ou l'ensemble des revendications 1 à 57, caractérisée en ce que la cuvette de support (24) est essentiellement composée de plastique.

59. Prothèse en selle selon la revendication 58, caractérisée en ce que la cuvette de support (24) est composée de polyéthylène.

60. Prothèse en selle selon l'une ou l'ensemble des revendications 1 à 59, caractérisée en ce que le coussinet (25) est composé de plastique.

61. Prothèse en selle selon l'une ou l'ensemble des revendications 1 à 60, caractérisée en ce que l'élément d'appui (41) est conçu comme un collet soutenant le corps (2) sur une grande surface sur le fémur (40).

62. Prothèse en selle selon l'une ou l'ensemble des revendications 1 à 61, caractérisée en ce que la cheville (11) présente dans la zone de son extrémité inférieure dans le sens vertical un accouplement (63) la raccordant à un outil de positionnement (64).

63. Prothèse en selle salon l'une ou l'ensemble des revendications 1 à 62, caractérisée en ce que l'axe transversal de la selle (30) est orienté dans la direction d'une force de pression sollicitant la cuvette de selle (7) dans la zone de sa liaison avec le cotyle (8) et agissant dans la direction de la résultante de force.

64. Prothèse en selle selon l'une ou l'ensemble des revendications 1 à 63, caractérisée en ce que la pièce intermédiaire (3) est conçue comme un élément adaptable, qui présente dans la direction d'un axe médian (81) de la cheville (11) une étendue pouvant être configurée.

65. Prothèse en selle selon l'une ou l'ensemble des revendications 1 à 63, caractérisée en ce que l'axe médian de la cheville (81) s'étend sensiblement dans la direction d'une résultante de force composée du poids et de la force musculaire.

66. Prothèse en selle selon la revendication 65, caractérisée en ce que l'axe médian de la cheville (81) présente un angle de 0 à 30 degrés par rapport à la verticale.

67. Prothèse en selle selon la revendication 66, caractérisée en ce que l'angle présente une valeur de 16 degrés environ.

68. Prothèse en selle selon l'une ou l'ensemble des revendications 1 à 67, caractérisée en ce que la goupille de guidage (34) s'étend dans la zone de son extrémité supérieure dans le sens vertical à travers un plan partant de la délimitation supérieure de la pièce intermédiaire (3) orientée vers la selle (1).

69. Prothèse en selle selon l'une ou l'ensemble des revendications 1 à 68, caractérisée en ce qu'au moins un élément d'amortissement (80) absorbant les contraintes de choc est prévu dans la zone de la pièce intermédiaire (3).

70. Prothèse en selle selon l'une ou l'ensemble des revendications 1 à 69, caractérisée en ce qu'un plan de rotation partant de la délimitation supérieure (27) de la pièce intermédiaire (3) orientée vers la selle (1) est sensiblement perpendiculaire à la résultante de force.

71. Prothèse en selle selon l'une ou l'ensemble des revendications 1 à 70, caractérisée en ce que la surface de coupe longitudinale (26) en forme de S présente une zone médiane d'étendue sensiblement linéaire, dont la longueur et l'inclinaison par rapport à l'horizontale présentent un rapport constant prédéterminé de manière fixe.
